Europäisches Patentamt

European Patent Office

Office européen des brevets

(19)

(11) Publication number: **0 282 192**

**A1**

(12) # EUROPEAN PATENT APPLICATION

(21) Application number: **88301480.5**

(22) Date of filing: **22.02.88**

(51) Int. Cl.4: **G01N 33/74** , G01N 33/543 , //G01N33/531,G01N33/94

(30) Priority: **09.03.87 US 23232**

(43) Date of publication of application:
**14.09.88 Bulletin 88/37**

(84) Designated Contracting States:
**AT BE CH DE ES FR GB GR IT LI LU NL SE**

(71) Applicant: **QUIDEL**
**11077 North Torrey Pines Road**
**La Jolla, CA 92037(US)**

(72) Inventor: **Kalid, Mohammed A.**
**12732 Monterey Cypress Way**
**San Diego California 92130(US)**
Inventor: **Katz, David H.**
**1775 La Jolla Rancho Road**
**La Jolla California 92037(US)**
Inventor: **Staples, Mark A.**
**11844 Arbor Lake Way**
**San Diego California 92131(US)**

(74) Representative: **Bond, Bentley George et al**
**HASELTINE LAKE & CO. Hazlitt House 28**
**Southampton Buildings Chancery Lane**
**London, WC2A 1AT(GB)**

(54) Competitive immunoassay for haptens using protein-hapten coupled solid phase.

(57) Apparatus for determining hapten comprising: a solid support surface; a coating of albumin or other spacer protein coupled to the solid surface; and a layer of the hapten to be determined covalently coupled to the surface of the albumin covalently, and a method for using the same is disclosed.

FIG. 1

# COMPETITIVE IMMUNOASSAY FOR HAPTENS USING PROTEIN-HAPTEN COUPLED SOLID PHASE

## Field of the Invention

This invention relates to immunochemistry, specifically to immunoassays for haptens, with particular and exemplary application in the determination of progesterone in animal husbandry for determining the optimum time for insemination.

## Background of the Invention

The determination of haptens in biological fluids from many sources is of considerable scientific, medical and, in some instances, economic and forensic value. It is important, for example, in some lines of scientific inquiry to determine the presence or absence of a particular hapten to determine a metabolic path or immunochemical phenomenon. Medically, it is important to determine the presence and/or amount of a particular hapten in fluids as one factor, often a key factor, in prescribing a particular course of treatment or in prescribing the dose level of particular drugs, etc. Forensically and medically, the determination of the presence, absence or level of concentration of various haptens is becoming extremely important as drug abuse pervades our society. It is of great economic and practical importance for cattle breeders to know when to inseminate a cow to induce pregnancy, and to be able to follow up on insemination to determine whether or not the inseminated cow is pregnant. While the present invention has wide application in the determination of haptens in biological fluids generally, the significant advantage of the invention is demonstrated in the determination of progesterone in bovine milk, as an illustrative and exemplary embodiment, and not as a limitation of the inventive concept involved. For example, the same principles and techniques can be used for determining the most advantageous time for insemination of other animals, and the technique has very general application as indicted hereinafter.

Progesterone content of milk drop is a good indicator of whether or not the animal, in this example a cow, is in heat or pregnant. During the follicular phase the progesterone content of milk from a non-pregnant cow remains at about 10 nanograms/milliliter or higher until the onset of estrous, "heat", when the progesterone level drops to under 5 ng/ml and often to less than 1 ng/ml during estrous, just before ovulation, signalling the ideal time for insemination to maximize the likelihood of impregnation and pregnancy. Progesterone levels remain high during pregnancy and in non-pregnant cows until the onset of estrous when the progesterone level drops again, as the cycle repeats. Thus, it is important, in the efficient use of semen, in artificial insemination, and the availability of a bull in natural insemination that insemination occur at the time when the cow is most susceptible to impregnation.

There are a number of techniques available for determining the level of progesterone in whole milk, most of which are expensive to carry out and have low sensitivity. Traditionally, radioimmunoassay (RIA) procedures have been used in progesterone determinations. Newer approaches to progesterone determination use enzyme linked immunoassay (ELISA) techniques.

Enzyme immunoassays have become widely known and described in numerous tests, treatises, scientific papers and patents since the pioneering work begun by Dr. Eva Engvall et al., Engvall, E. and Perlmann, P., Enzyme-linked immunosorbent assay (ELISA), Immunochem. 8:871-874, 1971, and the work of Schuurs and coworkers, see, e.g. Van Weemen Antigen-enzyme conjugates, FEBS Letters, 15:232-236, 1971, and several U.S. Patents naming Schuurs, et al., as inventors, see, e.g. U. S. Patents Nos. RE 31,006, 3,654,090, 3,839,153, 3,850,752, 3,862,302, 3,862,928, 3,879,262, and 4,016,043. Monoclonal antibodies in enzyme immunoassays are wellknown, U.S. Patent No. 4,376,110, and the work of Herzenberg and of Engvall, Rouslahti and Uotilla.

ENZYME IMMUNOASSAY, Ishikawa, M.D., Tadashi, Kawai, and Kiyoshi, Miyai, Editors, IGAKU-SHOIN, New York 1981 describes in considerable fundamental detail the principles and practices involved in enzyme immunoassays, including application to the immunoassay of progesterone, see Competitive Immunoassay of Progesterone, Ferdinand Dray and Claude Gross, ENZYME IMMUNOASSAY, supra, pp. 146-156, which cites Seeger et al, "An enzyme immunoassay of progesterone in horse plasma", J. Immunol. Methods, 28: 211-217, 1979, and other references which describe various RIA and EIA methods which may be applied in the determination of progesterone. Reference is also made to other texts and treatises in the field, such as IMMUNOCHEMICAL METHODS IN THE BIOLOGICAL SCIENCES: ENZYMES AND PROTEINS, Mayer, R.J. and Walker, J.H., Academic Press, New York 1980, and QUANTITATIVE ENZYME IMMUNOASSAY, Engvall, E. and Pesce, A.J., Blackwell Scientific Publications. London, (Scandinavian Journal of Immunology, 1978) and the reference cited therein for a comprehensive disclosure of the principles and usual practices involved in enzyme immunoassay. The principles of ELISAs are discussed by Belanger, L., Alternative Approaches in Enzyme Immunoassays, Scand. J. Immunol., Vol 8, Suppl. 7, 33-41, 1978 (Chapter 4 in QUANTITATIVE ENZYME IMMUNOASSAY, supra).

Many forms of solid supports to which one member of an immunochemical couple, e.g. antigen-antibody or hapten-antibody couple, have been disclosed. A common early form of solid support was a plate, tube or bead of polystyrene which was well-known from radioimmunoassay (RIA) work to bind certain immunological species. Filter paper, glass, various plastics (chemical polymers), and other solid support surfaces have been used for many years. Examples of such a system which used antibody (or antigen) coated polystyrene beads are described by Bohn, et al., in U. S. Patent No. 4,424,279, Jan. 3, 1984, and U.S. Patent No. 4,458,020, July 3, 1984, in which the coated balls are utilized in unique configurations. Katz et al have described avidin-biotin binding of immunogens, see U.S. Patent No. 4,458,020.

Protein chemistry is well developed and there are many well defined proteins available for use by the immunologist. One such protein, bovine serum albumin (BSA), which is used here simply as an example of the use of proteins generally, is a very well-known and widely used protein in the field of immunology and has been used in a great many applications. For example, Lehrer, U.S. Patent No. 4,351,824, used BSA to stabilize non-auto-agglutinating polystyrene; Rupchock, et al, U.S. Patent No. 4,366,243, used BSA to stabilize apoglucose oxidase compositions; Busby et al, U.S. Patent No. 4,317,879, used BSA in the preparation of a polytetrafluoroethylene-glucose oxidase electrode; and Cooper and O'Beirne, U.S. Patent No. 4,410,634, issued October 18, 1983, conjugated a hapten, e.g. digoxin, with BSA and then absorbed the resulting hapten-carrier conjugate on a solid phase surface. According to Cooper and O'Beirne, supra, immuno-reactive haptens are passively absorbed to convenient solid phases such as the surface of tubes or microtiter wells through the use of selected macromolecular carriers for the haptens. BSA, human serum albumin, egg albumin, and polylysine are mentioned as suitable carriers. Drugs, animal and plant hormones, antibiotics and pesticides are mentioned as haptens. Specific solid phases mentioned are plastic test tubes, microtiter wells and other containers of, for example, polystyrene, polyethylene or other plastic. In these and in most other applications of BSA in connection with haptens, this macromolecule is utilized basically as a carrier which otherwise does not enter into or significantly effect the immunochemical reactions involved. For example, insofar as is known, BSA has not been used as a spacing molecule which is covalently coupled to both a solid substrate and to a hapten, antibody or antigen.

Not withstanding the numerous approaches which have been made to developing more sensitive and more reliable, simpler enzyme immunoassays, most immunoassays suffer from various disadvantages in poor sensitivity, low reliability, difficulty in reading, overly complicated procedures, etc.; and there continues to be a need for improvements in simplicity, reliability, and sensitivity. It is, accordingly, an important feature of this invention to provide a highly sensitive, reliable and relatively simple enzyme immunoassay procedure, apparatus and kit which is particularly well suited to the determination of haptens and especially well suited to the determination of progesterone in milk.

Summary of the Invention

The present assay may be described in general terms as a protein-spaced-hapten competitive assay. The assay involves a solid support which may be in the form of beads, paper, disks, etc. The exemplary configuration is a dipstick having at least one pad of cellulose fibers, (e.g. filter paper), attached to it. (The support for the immunological reagents is the pad and the term "pad" is used here in reference to the exemplary pad depicted in the example, and also to encompass a solid support to which an immunological reagent is bound regardless of the particular configuration of the solid support.) The pad has bound to it a "spacer" protein, exemplary of which is BSA. While BSA is used as the example, other proteins can be used which (a) are or can be made soluble in water, (b) can be bound to the solid support, (c) can covalently bind a hapten, and (d) do not compete with or interfere with the immunochemical reaction of the hapten in the determination. Such proteins would be considered to be generally equivalent to BSA. Hapten of the type to be determined is bound to the spacer protein. The other reagents used in the present invention include a labelled antibody to the hapten and, if needed, a developer for the label on the antibody. Fluorescent, luminescent, radioisotopic and other labels can be used; however, by far the most convenient label presently available are the enzyme labels traditionally used in ELISA determinations of the type described in the background discussion above, along with suitable developers as also discussed above and in detail in the references cited. Thus, the assay of this invention may be described in general terms as a "protein-spaced-hapten competitive ELISA" type assay.

The invention is generaly similar to but is an improvement over a conventional competitive ELISA for haptens. In one form of prior art competitive ELISA's for haptens a predetermined amount of the hapten to be determined ("known hapten") has which was bound to a solid support competed with the hapten to be determined ("unknown hapten" for labelled antibody to the haptens. While this type of CELISA is immunochemically sound in theory, a great improvement has been discovered. It is this discovery that underlies the present invention. It has now been discovered that a very surprising, even striking increase in sensitivity can be achieved by binding a "spacer protein" between the solid support and the "known hapten". The solid support may be depicted in a general way by the following:

**DIPSTICK : PAD : SPACER PROTEIN : KNOWN HAPTEN**

wherein the colon ":" indicates a binding, either physical, chemical or immunochemical as appropriate. The pad may be physically or chemically affixed to the dipstick, the spacer protein is chemically bound to the pad, and the hapten is chemically or immunochemically bound to the spacer protein. In the description of the preferred embodiment, the solid support with the above attachments are referred to as a "dipstick"; however, other solid supports are also contemplated.

In carrying out the invention, the dipstick (or other solid support) having a hapten of the type to be determined bound to a spacer protein which, in turn, is bound to the solid support and labelled antibody are introduced into the sample. The "known hapten" on the dipstick competes with the "unknown hapten" (if any) in the sample for antigenic sites on the labelled antibody to the hapten. If there is no unknown hapten in the sample, then all of the labelled antibody will bind to the hapten which is coupled through the spacer protein on the solid support and, upon development, an intense color on the dipstick pad results. If there is a high level of unknown hapten in the sample, a substantial portion of the labelled antibody couples with the unknown hapten and remains in solution and a less intense color results on the dipstick pad. Thus, the color is inversely proportional to the concentration of the hapten in the sample.

The extraordinary increase in sensitivity resulting from the use of the spacer protein between the solid support, cellulose fibers for example, and the prior art makes the difference between an assay method and assay kit which can be used with high reliability in the atmosphere of the dairy farm and a method and kit which cannot.

The invention is embodied in apparatus for determining progesterone in milk, comprising a solid support surface, bovine serum albumin (or other spacer protein) covalently coupled to the solid support surface and progesterone (or other hapten) covalently coupled to the bovine serum albumin.

In one form, the invention embodies apparatus for determining hapten comprising a solid support surface, a coating of spacer protein covalently coupled to the solid surface, the spacer protein being covalently bound to hapten of the type to be determined and being characterized in that it (a) is or can be made soluble in water, (b) can be bound to the solid support, (c) can covalently bind a hapten, and (d) does not compete with or interfere with the immunochemical reaction of the hapten in the determination.

In a preferred form, the invention is embodied in an improved dipstick for determining immunological species of the type which comprises an elongate stick and at least one immunogen treated pad on the stick, the improvement wherein the dipstick is suitable for determining progesterone in milk. The dipstick usually includes two pads but always includes at least one dipstick pad consisting essentially of a large surface area pad formed of pad forming constituents selected from the group consisting of fibers, particles and a combination of fibers and particles bound together to form a porous layer. Bovine serum albumin is covalently coupled to the pad forming constituents, and progesterone is covalently coupled to the bovine serum albumin which is bound to the pad forming constituents.

Where two pads are used, the dipstick preferably includes a reference pad on the stick, the reference pad consisting essential of one member of a reference coupling pair covalently coupled to a large surface area pad formed of pad forming constituents selected from the group consisting of fibers, particles and a combination of fibers and particles bound together to form a porous layer, the reference coupling pair being characterized in that neither component of the reference coupling pair is reactive with either progesterone or antibodies to progesterone. The preferred reference coupling pair is avidin and biotin.

The invention may also be embodied in a kit for determining progesterone in milk. The kit would normally comprise a dipstick comprising an elongate solid supporting stick and at least two large surface area pads on the supporting stick, the pads being characterized in that one of said pads is a reference pad consisting essentially of the large surface area pad formed of pad forming constituents selected from the group consisting of fibers, particles and a combination of fibers and particles bound together to form a porous layer, with one member of a reference coupling pair bound to the pad forming constituents, the reference coupling pair being characterized in that neither member of the reference coupling pair is reactive with or interferes with the reaction of either progesterone or antibodies to progesterone. The other of said pads is a test pad consisting essentially of the large surface area pad formed of pad forming constituents selected from the group consisting of fibers, particles and a combination of fibers and particles bound together to form a porous layer, a coating of bovine serum albumin covalently coupled to said pad forming constituents, and progesterone coupled to the bovine serum albumin. The kit preferably includes a predetermined quantity of enzyme labelled anti-progesterone antibodies and a predetermined quantity of the other member of the reference coupling pair labelled with the same enzyme with which the antibodies are labelled.

## Brief Description of the Drawings

Figure 1 is a side view of the dipstick of the invention.

Figure 2 depicts one form of a kit embodying the principles of the invention.

Figure 3 is a block diagram depicting the steps of the method of the invention.

## Description of the Preferred Embodiment

The application of the principles of this invention is described with respect to the determination of the progesterone level in milk, simply as illustrative, and not as limiting the invention.

Progesterone is a $C_{21}$ steroid which is secreted during the luteal phase of the ovarian cycle and during pregnancy. It is formed as an intermediate in all steroid-secreting cells, and is produced and secreted as a hormone by the corpus luteum and by the placenta. Progesterone is necessary for the implantation of the fertilized ovum and for inhibition of uterine contraction in the maintenance of pregnancy. Progesterone stimulates the growth of the secretory glandular portions of the uterine and breast tissue, and is involved in inducing heat in cows during ovulation.

It is known that a non-pregnant cow will have a relatively constant level of at least 10 nanograms per milliliter (ng/ml) of progesterone in whole milk which she produces during the follicular phase of the ovulation cycle. Just prior to the time of ovulation, during estrous, the level of progesterone in the milk drops dramatically to less than about 3-5 ng/ml and usually to under 1 ng/ml. The drop in the progesterone content of the whole milk, if determined accurately, can be used to identify the onset of ovulation which occurs at about 21 day intervals and, hence, the time when insemination will most likely result in pregnancy. About 24 hours after ovulation, the progesterone content of the whole milk rises again. If insemination results in pregnancy, the progesterone remains high. If insemination does not result in pregnancy the progesterone concentration will drop again with the onset of estrous, indicating the need to repeat the insemination.

With this knowledge, an immunoassay procedure was developed to determined the progesterone level in bovine milk. In this particular application an exact quantitative determination is not necessary. It is, however, necessary to know whether the progesterone level is in the general range of about 10 ng/ml or higher or in the general range of 1-3 ng/ml or less. Thus, high sensitivity is necessary along with the ability to clearly distinguish between the two general levels. Since the test is designed to be used in the field by semi-skilled dairy workers, the results must be accurate and must be clearly positive or clearly negative, i.e. the cow was (a) in the ovulation cycle when insemination, either natural or artificial, would likely be most successful (i.e. in heat) or (b) pregnant or not in estrous.

In the early efforts, a dipstick with a reference pad and a test pad was prepared. The pads, before treating with immunochemically related reagents, were identical, being formed of constituents which resulted in a large surface area, e.g. fibers or particles, or a combination of fibers or particles, bound together into a layer or a body from which a layer was cut. A sheet of cellulose fibers, e.g. filter paper, works well but any large surface area pad generally as described can be used. The reference pad fibers were coated with avidin and progesterone was covalently bonded to the fibers of the test pad using prior art preparation and coupling reagents and procedures.

The assay for progesterone was carried out initially using dipstick to which anti-progesterone antibodies were bound to the reference pad and adding biotin labeled with alkaline phosphatase (ALP) and progesterone labelled with ALP to the milk sample and letting the mixture incubate. It was predicted that by using samples of known progesterone content from 1 to 10 ng/ml a typical competitive assay response curve would result, i.e. the color of the test pad would be inversely related to the amount of progesterone in the sample. It was found, however, that the sensitivity of the assay to progesterone was so low that it was impossible to develop a suitably sensitive and reliable test for use in the field.

Various efforts to overcome the problem were tried and ultimately the entire approach to making the test pad was abandoned and a new approach adopted.

It was discovered that if progesterone were covalently bound to a spacer protein, e.g. BSA or equivalent, and the spacer protein was covalently bound to the fibers or particles of the pad, a very high sensitive assay resulted which gave a very clear difference between a positive and a negative result. It is postulated that the BSA acts as a large (relative to the size of the antibody) spacer, positioning the progesterone antibody away form the fiber surface and exposing the determinant portion of the antibody for coupling with progesterone, or perhaps preventing steric or spacial hindrance.

The process of the invention, exemplified as a method for determining progesterone in milk, comprises the following steps. A reference pad consisting essentially of the large surface area pad to which one member of a reference coupling pair is bound, the reference coupling pair being characterized in that neither member of the reference coupling pair is reactive with or interferes with the reaction of either progesterone or antibodies to the hapten to be determined, e.g. progesterone, is inserted into the sample suspected of containing the hapten to be determined. A test pad consisting essentially of the large surface area pad, a coating of bovine serum albumin on the pad having hapten of the type to be determined covalently coupled to the bovine serum albumin is also inserted into the sample. A predetermined quantity of enzyme labelled anti-hapten antibody, e.g. anti-progesterone antibody, and a predetermined quantity of the other member of the reference coupling pair labelled with the same enzyme with which the anti-progesterone antibody is labelled are added to the sample. In the preferred embodiment biotin labelled with the ALP is added in an amount, empirically determined, to result in a color of the same intensity as results on the reference pad at the 5 ng/ml level. The sample is then incubated with the aforesaid reagents therein, the pads are separated from the sample and, preferably washed, and then the labelling enzyme is developed thereby to develop a color on the reference pad and on the test pad. The color of the test pad is compared visually or instrumentally with the color of the reference pad as a measure of the amount of progesterone, or other hapten, in the sample. If the test pad color is darker than the color of the reference pad, then the test indicates a low level of progesterone and tells the user that the cow is in estrous or the early phase of ovulation and that insemination is likely to result in impregnation. If the test pad is lighter than the reference pad, a high level of progesterone is present indicating that insemination would not be appropriate.

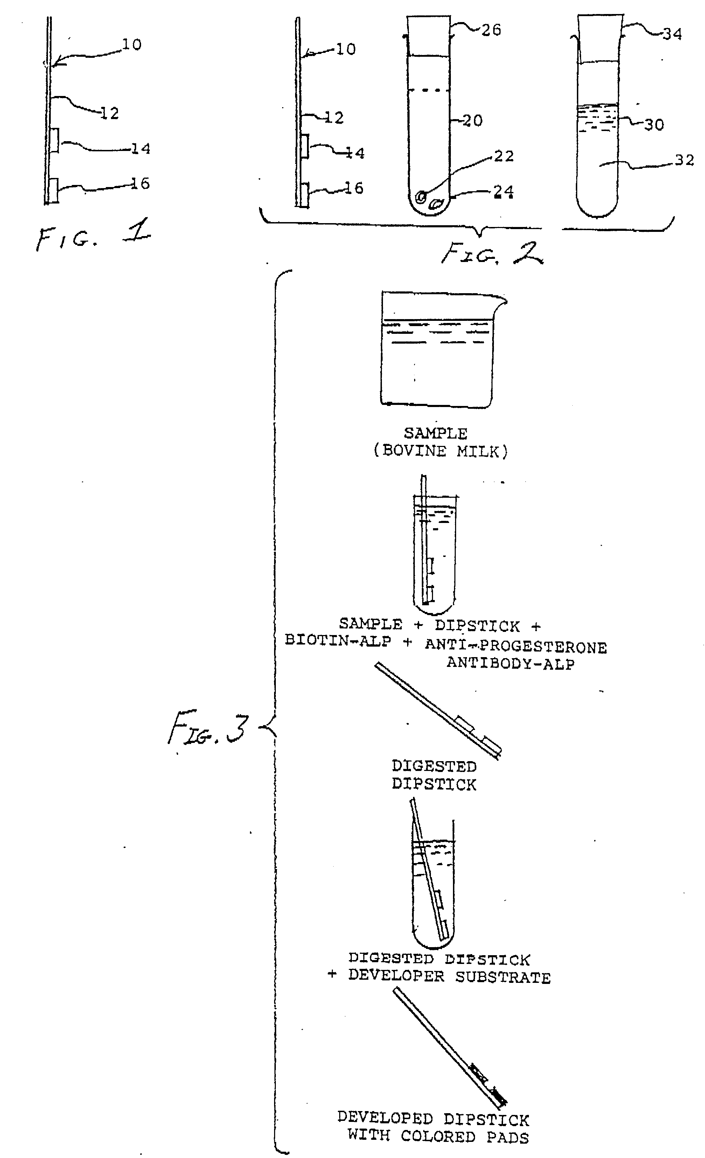

Referring now to the drawings, Figure 1 in particular, the invention is embodied in apparatus 10 for determining progesterone in milk, comprising a solid support surface 12, bovine serum albumin covalently coupled to the solid support surface and progesterone coupled to the bovine serum albumin.

In a more general sense, the invention embodies apparatus for determining hapten comprising a solid support surface, a coating of inert soluble spacer protein coupled to the solid surface and hapten of the type to be determined covalently coupled to the protein.

In a preferred form, the invention is embodied in an improved dipstick for determining immunological species of the type which comprises an elongate stick 10 and at least one immunogen treated pad, e.g. 12 on the stick, the improvement wherein the dipstick is suitable for determining progesterone in milk. The dipstick usually includes two pads 12 and 14 but always includes at least one dipstick test pad 12 consisting essentially of a large surface area pad formed of pad forming constituents selected from the group consisting of fibers, particles and a combination of fibers and particles bound together to form a porous layer. Bovine serum albumin is covalently coupled to the pad forming constituents, and progesterone is covalently coupled to the bovine serum albumin.

Where two pads are used, the dipstick preferably includes a reference pad 14 on the stick, the reference pad consisting essential of one member of a reference coupling pair covalently coupled to a large surface area pad formed of pad forming constituents selected from the group consisting of fibers, particles and a combination of fibers and particles bound together to form a porous layer, the reference coupling pair being characterized in that neither component of the reference coupling pair is reactive with either progesterone or antibodies to progesterone. A preferred reference coupling pair is avidin and biotin, but any reference couple which does not interfere with or immunochemically enter into the hapten - anti-hapten reaction can be used. For example, a rabbit IgG - anti-rabbit-ALP or a DNP - anti-DNP-ALP or any number of other reference couples can be used. Indeed, any non-interfering and non-reactive couple which can be determined in the 1 - 5 ng/ml level can be used as the reference couple.

A great many solid support materials have been used in immunochemistry. Among the more common of these are synthetic polymers (plastics) such as polystyrene, polymethylmethacrylate, polydivinylbenzene, etc., in the form of beads, tubes, plates, sticks, and in other configurations, and natural polymers, the most common natural polymer being cellulose in the form of a pad, disk, beads, paper, or some other convenient configuration. These and other immunologically inert solid supports may be used in this invention.

Enzyme amplification techniques and other techniques suitable for use with ELISA procedures generally may be used in connection with this invention without departing from the principle thereof. For example, a non-labelled mouse derived anti-progesterone antibody can be added to the sample to form the hapten-antibody couple and an excess of labelled goat anti-mouse antibody reacted with the solid phase. Since each mouse antibody binds two goat antibodies, the color intensity and color resolution can be approximately doubled, as compared with using a labelled anti-hapten antibody directly. This is, of course, but an example of the many ELISA related procedures which can be used within the principle of the invention.

The invention may also be embodied in a kit, depicted in Figure 2, for determining progesterone in milk. The kit would normally comprise a dipstick as described and may include two containers, such as test tubes **20** and **30**. The kit includes a predetermined quantity of enzyme labelled anti-progesterone antibodies **22** and a predetermined quantity of the other member of the reference coupling pair labelled with the same enzyme with which the anti-progesterone antibody is labelled, indicated at **24**. A conventional stopper **26** is normally provided. The kit may also include developer substrate **32** in tube **30** with a stopper **34**.

The invention is also embodied in the process or method for determining progesterone in milk, comprising the following steps, and making reference to Figure 3: A sample is obtained and added to the tube **20** thereby combining a reference pad consisting essentially of the large surface area pad to which one member of a reference coupling pair is bound, the reference coupling pair being characterized in that neither member of the reference coupling pair is reactive with or interferes with the reaction of either progesterone or antibodies to the hapten to be determined, e.g. progesterone, a test pad consisting essentially of the large surface area pad, a coating covalently coupled to said large surface area of bovine serum albumin having hapten to be determined covalently coupled to the bovine serum albumin is also inserted into the sample, a predetermined quantity of enzyme labelled anti-hapten antibody, e.g. anti-progesterone antibodies, and a predetermined quantity of the other member of the reference coupling pair labelled with the same enzyme with which the anti-progesterone antibody is labelled are added to the sample. The sample mixture is then incubated with the aforesaid reagents therein, the pads are separated from the sample and, preferably, washed, and then the labelling enzyme is developed thereby to develop a color on the reference pad and on the test pad. The color of the test pad is compared visually or instrumentally with the color of the reference pad as a measure of the amount of progesterone, or other hapten, in the sample.

The step-function increase in sensitivity in the assay of this invention as compared with the prior art methods was surprising, in that the increased sensitivity makes the difference between an assay which is suitable for field use and a assay which is unsuited to field use and of limited value in the controlled atmosphere of the research laboratory. This is of great practical and economic importance in the determination of progesterone in bovine milk, as it is not practical to operate a sophisticated laboratory in the atmosphere and circumstances of a dairy or feeding operation.

Although the presently contemplated best mode for carrying out the invention to its greatest advantage is as described above, the concept and principle of the invention is not so limited. In addition to the determination of progesterone in bovine milk in which application such striking results have been obtained, the method is applicable to the determination of progesterone in any milk or other fluid, and is of interest in determining other haptens. The determination of any hapten which may be found in body fluids or tissue which is soluble in or can be solubilized in a test solution is contemplated. Haptens generally, such as thyroxine, triiodothyronine, estriol, vitamin B12, and digoxin, steroids generally, including estradiols, testosterones, progesterone, oestrogens, cortisones, corticosterones, and other sterols as well as other haptens may be determined using the present invention. Among the haptens of interest are the "drugs of abuse". Drugs of abuse include a wide spectrum of drugs having a variety of physiological reactions when ingested or injected. Among the most common of these drugs of abuse are drugs which act as narcotics, hypnotics, sedatives, psychotogenic drugs, muscle relaxants, and nervous system stimulants. Particularly important drugs of abuse to which this invention is applicable, include opiates such as morphine, cocaine, amphetamines, barbiturates, phencyclidine, tetrahydrocannibinol (THC), methadone, Valium (oxazepam), and their metabolites.

Opiates which can be detected according to the principle of this invention include morphine, heroin, hydromorphone, oxymorphone, metopon, codeine, hydrocodeine, dihydrocodeine, dihydrohydroxycodeinone, pholocodeine, dextromethorphan, phenazocine and dionin.

Catacholamines, and the related compounds epinephrine, amphetamines, and other related compounds are also subject to determination by the apparatus and methods of this invention.

Barbiturates such as veronal, medinal, luminal, prominal, soneryl, nembutal, amytal, dial, phenadorn, seconal, evipan, phenobarbital and pentothal may be detected according to the principles of the present invention.

Cocaine, a drug now being very widely abused, and its analogs, and marijuana, also widely abused, may be detected by the present invention.

Tranquilizers such as meprobamate, and the benzdiazocycloheptanes are also abused and are subject to detection according to this invention.

Amino acids, peptides and polypeptides are also subject to detection according to this invention, as are other antigenic substances and organisms to which a specific monoclonal antibody can be produced.

Other substances which are subject to detection by the present invention include antibiotics such as penicillin, chloromycetin, actinomycetin, tetracycline, terramycin, nucleic acids or derivatives, such as nucleosides and nucleotides, steroids, hormones, insecticides, fungicides, bacteriocides and nematocides.

A wide variety of labeling enzymes that can be used in homogeneous type EIA have been reported in the literature. Typical of such enzymes are the oxidoreductases and hydrolases. The criteria for selecting those enzymes is well known, and has been discussed at some length by Rubenstein and Ullman, U.S. Patent No. 4,067,774. The criteria for selecting an enzyme for use in the present invention are quite different, however, than the criteria discussed by Rubenstein and Ullman. Particular enzymes suitable for use as labels in the present invention include: horseradish peroxidase (HRP), beta-galactosidase, acid phosphatase and alkaline phosphatase (ALP), which is used as exemplary of the many available enzymes.

Radioactive labels, a number of which are well known and generally used in the art, fluorescent labels and catalytic labels may be used. However, the preferred class of labels are the enzymes, as discussed, because of the simple and safe manner in which they can be employed without the need of specialized instrumentation or particular safety precautions.

In general, any immunologically inactive or immunologically non-interfering unconjugated or globular protein which is capable of being bound to a suitable solid surface can be used as a spacer. The serum albumins generally are most desirable because of they are well characterized and readily available. Human serum albumin and common mammal serum albumins, e.g. bovine serum albumin (BSA), are preferred simply on the basis of convenience and availability. In the forgoing example, BSA is used, but is used merely to exemplify the principle of the invention and not as a limitation. Only a very modest effort is necessary to assure that any particular soluble protein can be bound to the solid surface and does not bind or otherwise immunochemically react with the haptens or antibodies involved in the determination. Such proteins are referred to for simplicity as immunologically inert water soluble proteins.

It will be seen from the above discussion and examples that there are many variations which can be made as to steps, the order or sequence of the steps, the particular reagents, and the application of the process and apparatus, all without departing from the invention.

## Industrial Application

The scope of the invention is not limited by the preceding examples. Reference is made to the following claims which particularly point out and distinctly define the invention. ˙ Trademarks

## Claims

1. Apparatus for determining progesterone in milk, comprising:

(a) a solid support surface;

(b) bovine serum albumin coupled to the solid support surface; and

(c) progesterone covalently coupled to the bovine serum albumin.

2. Apparatus for determining hapten comprising:

(a) a solid support surface;

(b) a coating of albumin coupled to the solid surface; and

(c) a layer of the hapten to be determined covalently coupled to the surface of the albumin.

3. In a dipstick for determining immunological species of the type which comprises an elongate stick and at least one immunogen treated pad on the stick, the improvement wherein the dipstick is suitable for determining progesterone in milk, at least one dipstick pad consisting essentially of:

(a) a large surface area pad formed of pad forming constituents selected from the group consisting of fibers, particles and a combination of fibers and particles bound together to form a porous layer;

(b) a coating of bovine serum albumin coupled to the pad forming constituents; and

(c) progesterone covalently coupled to the bovine serum albumin.

4. The apparatus of Claim 3 further comprising:

(d) a reference pad on the stick, the reference pad consisting essential of one member of a reference coupling pair covalently coupled to a large surface area pad formed of pad forming constituents selected from the group consisting of fibers, particles and a combination of fibers and particles bound together to form a porous layer, the reference coupling pair being characterized in that neither component of the reference coupling pair is reactive with either progesterone or antibodies to progesterone.

5. The apparatus of Claim 4 wherein the reference coupling pair is avidin and biotin.

6. A kit for determining progesterone in milk, comprising:

(a) a dipstick comprising an elongate solid supporting stick and at least two large surface area pads on the supporting stick, the pads being characterized in that:

(i) one of said pads is a reference pad consisting essentially of the large surface area pad formed of pad forming constituents selected from the group consisting of fibers, particles and a combination of fibers and particles bound together to form a porous layer, one member of a reference coupling pair bound to the pad forming constituents, the reference coupling pair being characterized in that neither member of the reference coupling pair is reactive with or interferes with the reaction of either progesterone or antibodies to progesterone; and

(ii) one of said pads is a test pad consisting essentially of the large surface area pad formed of pad forming constituents selected from the group consisting of fibers, particles and a combination of fibers and particles bound together to form a porous layer, a coating of inert soluble spacer protein coupled to said pad forming constituents, and progesterone covalently coupled to the spacer protein;

(b) a predetermined quantity of enzyme labelled anti-progesterone antibody; and

(c) a predetermined quantity of the other member of the reference coupling pair labelled with the same enzyme with which the anti-progesterone antibody is labelled.

7. The kit of Claim 6 wherein the reference coupling pair consists essentially of avidin and biotin.

8. The kit of Claim 6 further comprising developer substrate for the enzyme label.

9. A method for determining hapten in solution, comprising:

(a) inserting into the sample in which hapten is to be determined:

(i) a reference pad consisting essentially of the large surface area pad to which one member of a reference coupling pair is bound, the reference coupling pair being characterized in that neither member of the reference coupling pair is reactive with or interferes with the reaction of either hapten or antibodies to hapten; and

(ii) a test pad consisting essentially of the large surface area pad, a coating coupled to said large surface area of inert soluble spacer protein, and a coating of hapten coupled to the spacer protein;

(b) adding to said sample a predetermined quantity of enzyme labelled anti-hapten antibody and a predetermined quantity of the other member of the reference coupling pair labelled with the same enzyme with which the anti-hapten antibody is labelled;

(c) incubating the sample with the aforesaid reagents therein;

(d) separating the pads from the sample;

(e) developing the labelling enzyme thereby to develop a color on the reference pad and on the test pad; and

(e) comparing the color of the test pad with the color of the reference pad as a measure of the amount of hapten in the sample.

10. The method of Claim 9 wherein the reference pad reference coupling pair consists essentially of avidin and biotin.

11. A kit for determining hapten in solution, comprising:

A. a dipstick having thereon:

1. a reference pad consisting essentially of pad to which one member of a reference coupling pair is bound, the reference coupling pair being characterized in that neither member of the reference coupling pair is reactive with or interferes with the reaction of either hapten or antibodies to hapten; and

2. a test pad consisting essentially of pad having a coating of spacer protein, the spacer protein being characterized in that the protein (a) is or can be made soluble in water, (b) can be bound to the solid support, (c) can bind a hapten, and (d) does not compete with or interfere with the immunochemical reaction of the hapten in the determination; and, hapten of the type to be determined bound to the coating;

B. a predetermined quantity of enzyme labelled antibody to the hapten to be determined; and

C. a predetermined quantity of the other member of the reference coupling pair labelled with the same enzyme with which the anti-hapten antibody is labelled.

12. Apparatus for determining hapten comprising:

(a) a solid support surface;

(b) a coating of protein coupled to the solid surface; and

(c) a layer of the hapten to be determined covalently coupled to the surface of the protein.

FIG. 1

FIG. 2

FIG. 3

SAMPLE
(BOVINE MILK)

SAMPLE + DIPSTICK +
BIOTIN-ALP + ANTI-PROGESTERONE
ANTIBODY-ALP

DIGESTED
DIPSTICK

DIGESTED DIPSTICK
+ DEVELOPER SUBSTRATE

DEVELOPED DIPSTICK
WITH COLORED PADS

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 101 912 (GENETIC DIAGNOSTIC CORP.) <br> * Whole document * | 1,2,12 | G 01 N 33/74 <br> G 01 N 33/543// <br> G 01 N 33/531 <br> G 01 N 33/94 |
| Y | | 3-11 | |
| Y | EP-A-0 203 238 (QUIDEL) <br> * Page 1, line 1 - page 2, line 16; page 8, line 23 - page 12, line 20; page 14, lines 8-13; claims; figures * | 3-11 | |
| X | EP-A-0 179 959 (MERCK FROSST CANADA INC.) <br> * Abstract; page 1, line 1 - page 2, line 14; page 23, lines 5-31 * | 2,12 | |
| X | US-A-4 235 960 (E.A.SASSE et al.) <br> * Column 3, lines 6-65; column 9, line 1 - column 10, line 15; claim 1 * | 12 | |
| X | EP-A-0 028 132 (DYNASCIENCES CORP.) <br> * Abstract; page 5, line 1 - page 7, line 8; claims * | 2,12 | TECHNICAL FIELDS SEARCHED (Int. Cl.4) |
| A | EP-A-0 161 107 (FARMOS-YHTYMÄ OY) <br> * Whole document * | 1-3,5,9,10,12 | G 01 N |

The present search report has been drawn up for all claims

| Place of search | Date of completion of the search | Examiner |
|---|---|---|
| THE HAGUE | 14-06-1988 | HITCHEN C.E. |

EPO FORM 1503 03.82 (P0401)